# EUROPÄISCHE PATENTSCHRIFT

(11) **EP 1 948 061 B1**
(45) Veröffentlichungstag und Bekanntmachung des Hinweises auf die Patenterteilung: **01.01.2014**
(21) Anmeldenummer: 06828885.1
(22) Anmeldetag: 30.10.2006
(51) Int. Cl.: A61B 19/00, A61C 13/00

(54) **PANORAMASCHICHTAUFNAHMEN ZUR IMPLANTATPLANUNG**
PANORAMIC TOMOGRAMS FOR IMPLANT PLANNING
ENREGISTREMENTS PANORAMIQUES STRATIFIES EN VUE DE LA PLANIFICATION D'UNE IMPLANTATION

(30) Priorität: 14.11.2005 DE 102005054571; 22.11.2005 DE 102005055896
(43) Veröffentlichungstag der Anmeldung: 30.07.2008
(73) Patentinhaber: siCAT GmbH & Co. KG, 53177 Bonn (DE)
(72) Erfinder: HANSSEN, Nils, 53111 Bonn (DE); KEEVE, Erwin, 53115 Bonn (DE); RITTER, Lutz, 53332 Bornheim (DE)
(74) Vertreter: Braun-Dullaeus, Karl-Ulrich
(86) Internationale Anmeldenummer: PCT/EP2006/010415
(87) Internationale Veröffentlichungsnummer: WO 2007/054219

(56) Entgegenhaltungen:
- US-B1- 6 493 415
- VERLY J G ET AL: "3D nonrigid registration and multimodality fusion for image-guided neurosurgery" INFORMATION FUSION, 2003. PROCEEDINGS OF THE SIXTH INTERNATIONAL CONFERENCE OF JULY 8-11, 2003, PISCATAWAY, NJ, USA,IEEE, Bd. 2, 8. Juli 2003 (2003-07-08), Seiten 852-859, XP010675635
- IMPLANT3D QUICK REFERENCE GUIDE, [Online] 2003, Seiten 1-35, XP002420626 Gefunden im Internet: URL:http://www.implant3d.com/Implant3D.pdf > [gefunden am 2007-02-16]
- VERSTREKEN K ET AL: "AN IMAGE-GUIDED PLANNING SYSTEM FOR ENDOSSEOUS ORAL IMPLANTS" IEEE TRANSACTIONS ON MEDICAL IMAGING, IEEE SERVICE CENTER, PISCATAWAY, NJ, US, Bd. 17, Nr. 5, Oktober 1998 (1998-10), Seiten 842-852, XP011035786 ISSN: 0278-0062
- PAREL S M ET AL: "Interactive imaging for implant planning, placement, and prosthesis construction" JOURNAL OF ORAL AND MAXILLOFACIAL SURGERY, SAUNDERS, PHILADELPHIA, PA, US, Bd. 62, September 2004 (2004-09), Seiten 41-47, XP004590902 ISSN: 0278-2391
- E. KEEVE, N. HANSSEN, J. HEY, T. JANSEN, B. VON RYMON-LIPINSKI: "ImPlan: A Planning Environment for Computer-Aided Dental Implant Placement" CAESAR ANNUAL REPORT, [Online] Juli 2005 (2005-07), Seiten 200-201, XP002420627 Gefunden im Internet: URL:http://www.caesar.de/uploads/media/AR0 4_final_web.pdf> [gefunden am 2007-02-16]

## Beschreibung

Die vorliegende Erfindung betrifft ein Verfahren zur zweidimensionalen Darstellung von Volumendaten eines Körperteiles, insbesondere des Kiefers, eines Patienten, wobei die Volumendaten mittels eines tomographischen Aufnahmegerätes, insbesondere eines "cone beam CT" Gerätes, aufgenommen sind, wobei den Volumendaten dreidimensionale Planungsdaten zugeordnet sind, die insbesondere geometrische Daten eines einzusetzenden Implantats, wie dessen Lage, Ausrichtung., Länge und Durchmesser, aufweisen, wobei in den Volumendaten eine Projektionsschicht definiert wird, die insbesondere durch eine Abwicklung als Panoramaaufnahme in der Darstellungsebene dargeboten wird.

Dentale Implantate werden bekanntermaßen anhand von Röntgenaufnahmen geplant, die über drei Dimensionen, insbesondere mit einem Cone Beam Gerät, aufgenommen sind. Dazu werden aus den Volumendaten verschieden orientierte Schichtansichten errechnet und dargestellt, in denen sich die Lage der virtuellen Implantate festlegen lässt. Zum Zwecke der guten Übersichtlichkeit, wird aus den Volumendaten eine Panoramaaufnahme in der Art eines dem Zahnarzt bekannten Orthopantomogramms (OPG) erzeugt. Eine solche Panoramaaufnahme gewährt einen Überblick über den kompletten Zahnstatus des Patienten. Dabei kann die Form der Kurve (Panoramalinie), entlang derer die Panoramaaufnahme erstellt wird, innerhalb einer parallel zum Boden verlaufenden Ebene, frei definiert werden.

Bei den Panoramaaufnahmen respektive bei deren Darstellung ist zwischen einer durch Vertikalverschiebung der Panoramalinie erzeugten Panoramaschicht und einer Panoramaprojektion, die durch die mittelwertbildende Summierung der Absorptionskoeffizienten des Volumens senkrecht zur einer Panoramaschicht entsteht, zu unterscheiden. Eine solche Panoramaprojektion, also die Mittelung über viele Panoramaschichten, simuliert die Darstellung eines OPG, wobei die Tiefeninformation wegen der Summierung über alle Schichten fehlt.

Bei heutigen Planungssystemen können in der Panoramaaufnahme auch die im Volumendatensatz platzierten und darin durch ihre Planungsdaten definierten Implantate angezeigt werden. Eine solche Darstellung ermöglicht dem Zahnarzt eine schnelle Beurteilung der Situation betreffend die Anzahl und die Lage der Implantate im Kiefer des Patienten. Mit einem Wechsel der Darstellung zu einer korrespondierenden Panoramaschicht können die Schnitte der Panoramaschicht mit den Implantaten angezeigt werden. Zudem können Panoramaprojektion und Panoramaschicht zum Anordnen der Implantate genutzt werden. Dies kann dadurch geschehen, dass der Nutzer eine Stelle in derPanoramaprojektion oder der Panoramaschicht anklickt und damit das virtuelle Implantat an dieser Stelle der Panoramaschicht im 3D-Volumen anordnet. Dabei wird die Implantatachse bislang stets vertikal ausgerichtet.

Die bekannten Panoramaaufnahmen sind jedoch nur bedingt zur Beurteilung der wirklichen Lage und Orientierung der virtuellen Implantate im Knochen geeignet, da geneigt liegende Implantaten verzerrt darstellt respektive schief angeschnitten werden. Diese Darstellungen dienen daher in erster Linie der ersten Übersicht über die Anatomie und nicht der geplanten Implantate. Da die Panoramaschicht nicht unbedingt alle Implantate schneidet, sind zudem auch nicht alle Implantate in der Panoramaschicht sichtbar. Durch eine Neigung werden die Implantate schief geschnitten und erscheinen in der Panoramaschicht als Ellipsen.

Aus der DE 44 28 331 A1 ist eine Strahldiagnoseeinrichtung zur Erstellung solcher Panorama-Schichtaufnahmen bekannt, bei denen sich die Panoramaschichten in gewissem Maße an den anatomischen Gegebenheiten des Patienten ausrichten können. So können die Panoramaschichten insbesondere an der Neigung der Zähne ausgerichtet sein. Auch der Einsatz solcher an die Anatomie angepasster Panoramaschichten löst die Probleme bei der Darstellung der geplanten Implantate nicht.

Aus Verly J. G. et al, "3D nonrigid registration and multymodality fusion für image-guided neurosurgery" Information fusion, 2003, Proceedings of the 6th international conference of Huly 8-11, 2003 Piscataway, Bd. 2, 8. July 2003, Seiten 842-852, ist es bekannt, unterschiedliche Bilddatensätze zu registrieren. So wird ein vor einer Operation erstellter Bilddatensatz eines Patienten mit einem während einer Operation erstellten Bilddatensatz registriert, um Unterschiede zwischen den Bilddatensätzen, beispielsweise Operationsinstrumente kenntlich zu machen.

Aus IMPLANT3D QUICK REFERENCE GUIDE, (Online) 2003, Seiten 1-35 ist ein Darsteltungsverfahren bekannt, das sich einer an Planungsdaten orientierten Projektionslinie bedient.

In VERSTREKEN K ET AL.: "AN IMAGE-GUIDED PLANNING SYSTEM FOR ENDOSSEOUS ORAL IMPLANTS" IEEE TRANSACTIONS ON MEDICAL IMAGING, IEEE SERVICE CENTER, PISCATAWAY, Bd. 17, Nr. 5, Oktober 1998, Seiten 842-852 werden Darstellungsebenen entlang einer Kurve ausgerichtet, die durch einen Kieferbogen verläuft. Die Ausrichtung verläuft jeweils im rechten Winkel zu Tangenten, welche die Kurve berühren.

Das in PAREL S M ET AL: "Interactive imaging for implant planning, placement, and prosthesis construction" JOURNAL OF ORAL AND MAXILLOFACIAL SURGREY, SAUNDERS, PHILADELPHIA, PA, US, Bd. 62, September 2004, Seiten 41-47 offenbarte Darstellungsverfahren ist ähnlich. Dabei wird eine Darstellung des Kiefers anhand einer Schnittansicht erzielt, die punktförmig an einer Kieferbogenlinie ausgerichtet ist und den Kiefer im rechten Winkel zum Kieferbogen schneidet.

Die US 6,493,415 beschreibt ein Verfahren zur Ermitting einer vertikal gekrümmten Panoramaschicht. Jeder reals vorhandene Zahn wird dabei imwesentlichen entlang seiner vollen Länge dargestellt. In der Ausrichtung dieser Panoramaschicht werden geplante Implantate nicht berücksichtigt.

Die Aufgabe der Erfindung liegt nun darin, ein gattungsgemäßes Verfahren zu schaffen, das sich einfach umsetzen lässt und mit dem erreicht werden kann, dass der behandelnde Arzt während der Planung von Implantaten stets über eine optimale zweidimensionale Darstellung des Kiefers unter Berücksichtigung der aktuellen Implantatplanung verfügt. Zudem ist es Aufgabe der Erfindung, eine Vorrichtung zur Umsetzung des Verfahrens zu schaffen.

Diese Aufgabe wird durch das Verfahren nach Anspruch 1 gelöst. Besonders vorteilhafte Ausführungsformen sind in den Unteransprüchen genannt.

Der Grundgedanke der Erfindung liegt darin, eine Panoramadarstellung anhand einer zugrundeliegenden Panoramaschicht zu erzeugen, die zumindest lokal an einer Symmetrieachse des geplanten "virtuellen" Implantats respektive des entsprechenden für dieses Implantat vorgesehenen Eingriffskanales ausgerichtet ist. Die Darstellung orientiert sich damit nicht mehr, wie bislang, an einer mehr oder weniger willkürlichen Vorgabe oder an anatomischen Verhältnissen, sondern an der Lage eines geplanten Implantats. Der Verlauf der darzustellenden Projektionsschicht wird somit an den Planungsdaten respektive an einer an den Planungsdaten angepassten gebogenen Fläche angepasst. Dabei kann diese Ausrichtung der Panoramaschicht lokal auf den Ort des Implantates beschränkt sein und sich außerhalb dieses Bereiches den anatomischen Verhältnissen anpassen.

Erfindungsgemäß weist die Panoramaschicht somit am Ort des Implantats gewissermaßen eine "Verwerfung" auf und verläuft in dieser Zone der Verwerfung durch eine vom Implantat vorgegebene Fläche. So kann das Implantat vollständig dargestellt werden, wobei der besondere Vorteil dieser Vorgehensweise darin besteht, dass einer solchermaßen kalibrierten Darstellung besonders gut anzusehen ist, wie das Implantat in der umgebenden Anatomie angeordnet ist. Damit lassen sich Planungsfehler, wie das Eindringen in kritische Bereiche, wesentlich besser als bislang vermeiden.

Nachdem der Arzt anhand der ersten Panoramaaufnahme festgestellt hat, dass die Implantate noch richtig ausgerichtet sind, ist es vorteilhaft, wenn er deren Lage "online" am Bildschirm, beispielsweise durch Mausklick, korrigieren kann. Wenn er das tut, ist es erfindungsgemäß vorgesehen, die der Panoramaaufnahme zugrundeliegende Panoramaschicht automatisch der Änderung der Position, der Neigung, des Durchmessers oder der Länge des virtuell bereits eingesetzten Implantats anzupassen. Aus der so angepassten Panoramaschicht kann jeder Zeit wieder eine Panoramaprojektion über mehrere Panoramaschichten generiert werden.

Im Falle der Planung dentaler Implantate ist es vorteilhaft, wenn eine erste Panoramaschicht, die als Referenz für die spätere Summierung dienen kann, durch definierte Punkte in dem geplanten Implantat verläuft. Als solche Punkte bieten sich ein zur Kaufläche hin gerichteter (okklusaler) und ein zur Wurzel hin gerichteter (apikaler) Punkt an, wobei der okklusale und der apikale Punkt insbesondere von den Durchtrittspunkten der Symmetrieachse durch die Deckflächen eines durch das Implantat gebildeten Zylinders oder Konus definiert wird.

In einem ersten Schritt des Verfahrens ist es vorteilhaft, wenn der Verlauf einer ersten Panoramaschicht mit einem der bekannten Verfahren an den Verlauf des Kieferknochens oder an den Zähnen angepasst wird. Diese erste anatomisch orientierte Panoramaschicht wird dann entsprechend der Planungsdaten eines geplanten Implantates rechnerisch an der entsprechenden Stelle lokal gewölbt, um das geplante Implantat vollständig mit einzubeziehen. Generell kann aus der Panoramaschicht durch Summation über mehrere parallele Schichten wieder eine Panoramaprojektion erzeugt werden.

Um einen besonders realistischen Überblick über die von der Anatomie und den Implantaten gebildeten Gesamtsituation zu erhalten, ist es besonders vorteilhaft, wenn diese Panoramaschicht durch die okklusalen und die apikalen Punkte aller in dem betrachteten Volumen befindlichen Implantate verläuft. Dabei verwirft sich die Panoramaschicht derart, dass alle Implantate auf der von ihr gebildeten Ebene liegen. An den anderen Stellen ist es vorteilhaft, die Panoramaschicht an die anatomischen Verhältnisse, beispielsweise an gewisse Symmetrieachsen der Zähne, anzupassen.

Im Gegensatz zur "klassischen" Panoramaprojektion wird erfindungsgemäß somit nicht nur eine allgemeine Darstellung des Zahnstatus erstellt, sondern es ist eine zuverlässige Bewertung der Lage aller geplanter Implantate im Kieferknochen des Patienten möglich. Anders als die klassische Panoramaschicht verfügt die speziell angepasste Panoramaschicht nun zusätzlich über Verwerfungen an der Stelle eines Implantats. An Stellen, an denen kein Implantat geplant ist, ist es vorteilhaft, die Lage der Panoramaschicht zu interpolieren, um einen "weichen" Übergang sicherzustellen. Dabei ist es zudem vorteilhaft, wenn der Interpolationsalgorithmus austauschbar ist und kann an den "implantatlosen" Stellen durch zusätzliche Information beispielsweise aus dem Volumendatensatz parametrisiert wird. Dadurch können in der Panoramaschicht sowie in der Panoramaprojektion alle Implantate gleichzeitig und verzerrungsfrei dargestellt werden.

Wenn ein neues Implantat in die Panoramaschicht oder die Panoramaprojektion eingesetzt wird, so passt sich das Implantat zunächst vorteilhafterweise an die bislang definierte Schicht durch eine Parametrisierung an. Wenn demnach ein Implantat beispielsweise durch einen Klick mit dem Cursor auf dem Bildschirm zwischen zwei im Oberkiefer befindlichen Implantaten platziert wird, so bekommt dieses die interpolierte Position und Neigung der benachbarten Implantate. Ist es einmal auf diese Art und Weise eingesetzt, passt sich wiederum die Schicht bei jeder Positions-, Neigungs-, Längen, bzw. Durchmesseränderung an das Implantat an.

Eine derart an die Lage der Implantate angepasste Panoramaaufnahme hat einige Vorteile: Zunächst können alle Implantate innerhalb derselben Panoramaschicht entlang einer von ihnen vorgegebenen Achse, insbesondere ihrer Hauptachse, dargestellt werden. Insbesondere können alle Implantate innerhalb derselben Panoramaschicht entlang ihres größten Querschnitts dargestellt werden. Wie schon gesagt, können alle Implantate innerhalb derselben Panoramaprojektion verzerrungsfrei dargestellt werden. Dabei ist es vorteilhaft, dass die Anatomie entlang des größten Querschnitts der Implantate ohne Interaktion mit der Schicht an allen Implantaten gleichzeitig bewertet werden kann. Durch Einschränkung des Summierungsbereiches, der sich insbesondere über die Stärke des Implantats erstreckt, kann die Anzeige der Panoramaprojektion auf die anatomischen Bereiche beschränkt werden, die relevant für die Bewertung der voraussichtlichten Implantatfestigkeit sind. Mit der Erfindung kann eine gezielte Beurteilung der Knochenqualität in der Nähe der Implantate durchgeführt werden. Da die Panoramaschicht Position und Neigung der bereits vorgesehenen Implantate interpoliert, können diese Parameter für weitere hinzuzufügende Implantate von den benachbarten Implantaten abgeleitet werden.

Nachfolgend wird die Erfindung anhand der Figuren 1 bis 4 näher erklärt. Dabei zeigen:
- **Figur 1**: das Schema zur Entwicklung einer Panoramaschicht,
- **Figur 2**: Anpassungen der Panoramaschicht an ein Implantat,
- **Figur 3**: Anpassungen der Panoramaschicht an ein Implantat und
- **Figur 4**: die Summation über mehrere Panoramaschichten.

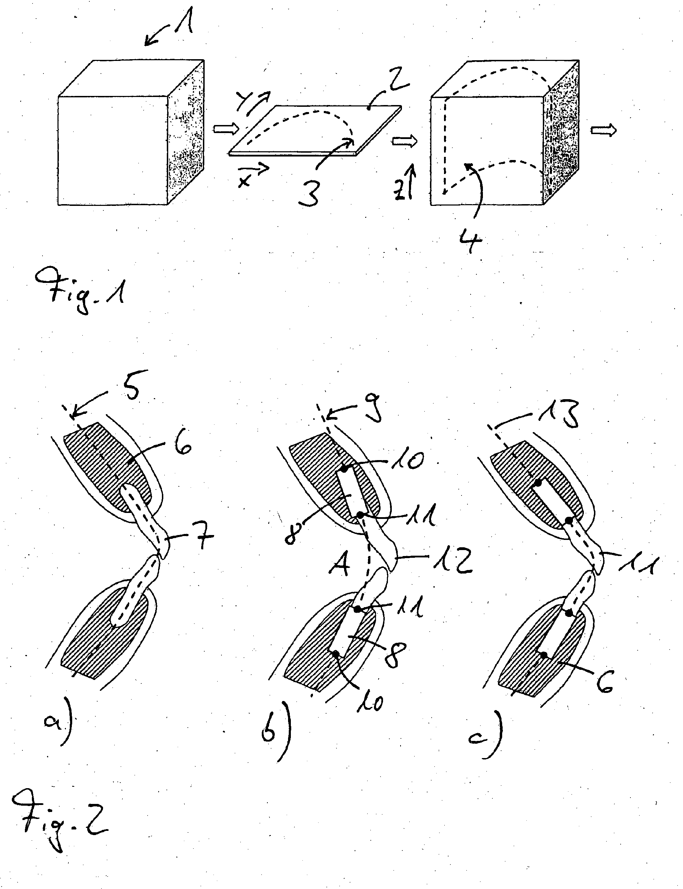

In Figur 1 ist ein Satz dreidimensionaler Volumendaten 1 in Form eines Quaders dargestellt. Innerhalb der Volumendaten 1 wird eine x-y-Ebene 2 herausgegriffen, in der eine Panoramalinie 3, die hier die Biegung eines Kiefers nachvollzieht, festgelegt wird. Durch eine Verschiebung in z-Richtung, die hier der Einfachheit halber nur in vertikaler Richtung erfolgt, entsteht eine Panoramaschicht 4. Diese "dünne" Panoramaschicht 4 kann auf unterschiedliche Weise, insbesondere durch Abwicklung, in eine Ebene transformiert werden und stellt dann eine Panoramaaufnahme dar. Eine Panoramaprojektion entsteht dann durch die Summierung einer Vielzahl von übereinanderliegenden derartigen Panoramaschichten 4. Dabei ist es vorteilhaft, die Anzahl der Schichten, über-die summiert wird, auf die Dicke der gesetzten Implantate zu beschränken.

Die initiale Ausrichtung dieser ersten Panoramaschicht 4 kann mit verschiedenen Ansätzen geschehen. Sie kann, wie in Figur 1 gezeigt, durch eine "klassische" Panoramaschicht, die durch manuelles Einzeichnen einer Panoramalinie in eine Schrüttebene entsteht, erfolgen, wobei diese Panoramaschicht zunächst keine vertikale Krümmung aufweist. Nachdem virtuelle Implantate eingefügt wurden, passt sich die Krümmung der Schicht schrittweise an. Es ist auch möglich, die initiale Panoramaschicht entlang der Kieferbögen und der Zahnwurzeln bzw. der Zähne mit vertikaler Krümmung festzulegen oder eine Ausrichtung anhand eines zuvor gespeicherten "Durchschnittskiefers" vorzunehmen. Die initiale Schicht kann auch aufgrund von Patientenparametern wie z.B. Alter und Geschlecht geschätzt werden. Auch Mischformen der dargestellten Methoden sind möglich. Die initiale Ausrichtung der Panoramaschicht 5 anhand anatomischer Parameter des Kiefers 6 und der Zähne 7 ist in Figur 2a gezeigt. Sind, wie hier, noch keine Implantate in dem Volumendatensatz platziert, nimmt die Panoramaschicht 5 eine vordefinierte Form an. Erst nach dem Platzieren der Implantate im Datensatz passt sich die Schicht 5 an diese an.

Aufgrund der Panoramadaten werden nun Implantate 8 geplant und ihre Lage im Kiefer festgelegt(Figur 2b). Ausgehend von der initialen Panoramaschicht 5 berechnet der Computer eine anhand der Implantatdaten modifizierte Panoramaschicht 9, die er durch die apikalen 10 und die okklusalen Punkte 11 der Implantate 8 legt. Diese Panoramaschicht 9 wird nun in die Ebene projiziert und auf dem Bildschirm dargestellt. Der Zahnarzt stellt aufgrund der anatomischen Gegebenheiten im Umfeld der Implantate und hier auch wegen der in der Panoramaaufnahme nicht dargestellten Zähne 12, die im Bereich A außerhalb der Panoramaschicht 9 liegen, fest, dass die Implantate 8 schief liegen. Dann kippt er die geplanten Implantate 8 durch einen Mausklick auf dem Bildschirm an und korrigiert deren Lage solange, bis die Panoramaschicht 13 (Figur 2c) durch die Zähne 11 verläuft und die Implantate korrekt im Kiefer 6 platziert sind.

Die neuen Implantate können durch einen Klick mit der Maus in die abgewickelte Panoramaprojektion bzw. die Panoramaschicht gesetzt werden. Dabei richten sich die so gesetzten Implantate nach der bereits durch die anderen Implantate definierte Panoramaschicht aus. Dabei wird auch die Neigung der Implantate berücksichtigt. Der Benutzer klickt dabei an die Stelle, an der der okklusale Punkt des Implantates zu liegen kommen soll. Je mehr Implantate sich bereits in der Planung befinden, desto genauer wird die so durchgeführte Ausrichtung von neu hinzugefügten Implantaten. Wird die Länge des Implantates nachträglich geändert, verschiebt sich nur der apikale Punkt des Implantates; der okklusale Punkt ändert dabei seine Position nicht.

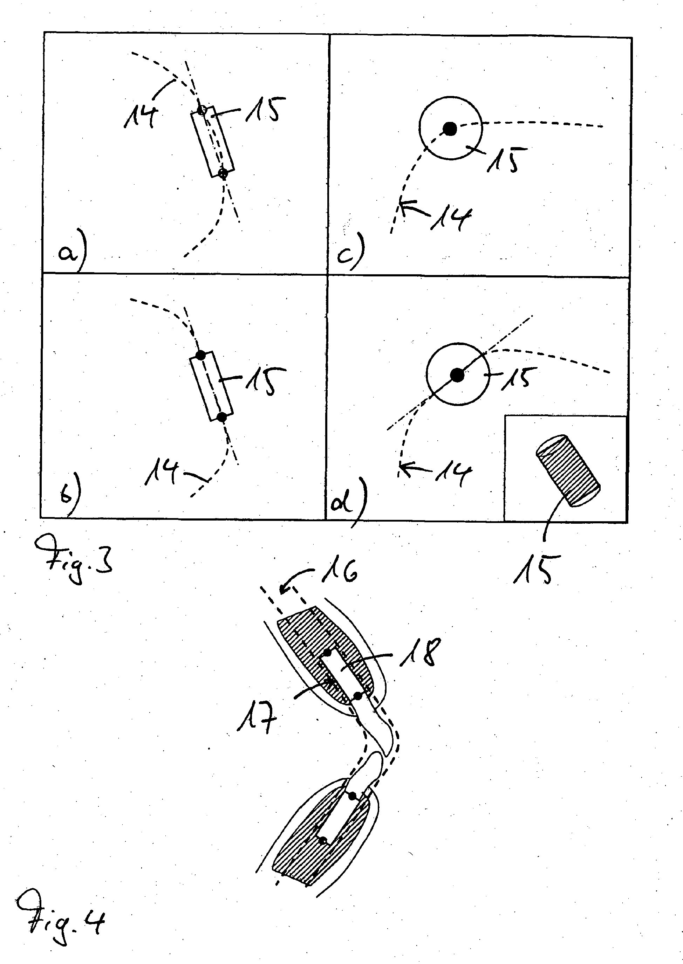

In Figur 3 sind verschiedene Arten der Anpassung der Panoramaschicht 14 an ein zylindrisches Implantat 15 gezeigt. Dieses Implantat 15 ist in der linken Spalte in Seitenansicht gezeigt. Im Fall a) wird nur der okklusale 16 und der apikale Punkt 17 des Implantates von der Panoramaschicht 14 durchlaufen. In der Draufsicht nach Figur 3c und der Seitenansicht nach Figur 3b wird die komplette Implantatachse zwischen dem okklusalem und dem apikalem Punkt durchlaufen, wobei die Panoramaschicht 14 innerhalb des Implantats noch horizontal gekrümmt sein kann. In der Draufsicht nach Figur 3d schneidet die Panoramaschicht 14 das Implantat 15 entlang seines größten Querschnittes.

Wie in Figur 4 dargestellt, kann die Erstellung der Panoramaprojektion, also der gemittelten Summe über mehrere Schichten, automatisch auf einen Bereich 16 senkrecht zur Panoramaschicht 13 (Figur 3c) beschränkt werden, der sich in unmittelbarer Nähe der Implantate befindet. So erhält die Panoramaprojektion nur die für die Bewertung der Implantatlage notwendigen Informationen über die angrenzenden anatomischen Strukturen und wird nicht durch unbedeutende Information überlagert. Durch einen geeigneten Sicherheitsbereich 17 werden die Bereiche, die unmittelbar vor und hinter den Implantaten 18 liegen, einbezogen werden. Dies ist auch für unterschiedlich dicke Implantate realisierbar, indem die Dicke des Summierungsbereiches an die Dicke der Implantate entsprechend angepasst wird. Eine solche Summation über die Breite der Implantate ist vorteilhaft, da damit nur die von den Implantaten betroffene Anatomie in der Panoramaaufnahme dargestellt wird.

Um den Knochen vor und hinter den Implantaten detailliert beurteilen zu können, kann die Schicht als Ganzes parallel zu den Implantatachsen nach "vorne und hinten" verschoben werden. Dabei wird die Schicht an jedem Punkt um die gleiche Strecke entlang ihrer Normalen verschoben. Dadurch können durch eine globale Verschiebung der Schicht alle Implantate gleichzeitig bewertet werden, egal, wie viele Implantate sich in der Planung befinden. Dadurch kann eine wesentlich schnellere, da parallele, Bewertung des Knochens im Umfeld der Implantate vorgenommen werden.

## Patentansprüche

1. Verfahren zur zweidimensionalen Darstellung von Volumendaten (1) eines Kiefers eines Patienten, wobei die Volumendaten (1) mittels eines tomographischen Aufnahmegerätes aufgenommen sind, wobei den Volumendaten dreidimensionale Planungsdaten zugeordnet sind, die geometrische Daten mindestens eines einzusetzenden Implantats (8) aufweisen, wobei in den Volumendaten eine darzustellende Panoramaschicht (9) definiert wird, die als Panoramaaufnahme in der Darstellungsebene dargeboten wird,
und zunächst eine intitiale Panoramaschicht (5) festgelegt wird, aufgrund derer Implantate und deren Lage im Kiefer festgelegt werden,
**dadurch gekennzeichnet,**
**dass** dann ausgehend von der initialen Panoramaschicht (5) anhand der Lage und der geometrischen Daten der Implantate die darzustellende Panoramaschicht (9) berechnet wird, die durch apikale und okklusale Punkte (10, 11) der Implantate gelegt wird, wobei die darzustellende Panoramaschicht (9) zumindest lokal horizontal und vertikal gekrümmt wird,
**dass** die Ausrichtung der darzustellenden Panoramaschicht einer Positions-, Neigungs-Längen- oder Durchmesseränderung eines Implants automatisch durch einen auf einem Computer laufenden Algorithmus angepasst wird.

2. Verfahren nach Anspruch 1,
**dadurch gekennzeichnet,**
**dass** der Verlauf der darzustellenden Panoramaschicht (9) an einer an den Planungsdaten angepassten gekrümmten Fläche ausgerichtet wird.

3. Verfahren nach Anspruch 1 oder 2,
**dadurch gekennzeichnet,**
**dass** die darzustellende Panoramaschicht (9) an allen im in den Planungsdaten befindlichen, virtuellen Implantaten (8) ausgerichtet wird.

4. Verfahren nach einem der vorherigen Ansprüche,
**dadurch gekennzeichnet,**
**dass** neue Implantate (8) durch eine Eingabe am Bildschirm, insbesondere durch Mausklick, in der darzustellenden Panoramaschicht (9) oder der Panoramaprojektion eingesetzt werden.

5. Verfahren nach Anspruch 4,
**dadurch gekennzeichnet,**
**dass** die Position und/oder die Neigung der hinzugefügten Implantate (8) automatisch an der durch bereits gesetzte Implantate (8) definierten Panoramaschicht (4) ausgerichtet wird.

6. Verfahren nach einem der vorherigen Ansprüche,
**dadurch gekennzeichnet,**
**dass** bei der Erstellung einer Panoramaprojektion, also der gemittelten Summe über mehrere Panoramaschichten auf einen Summierungsbereich, die Dicke eines Summierungsbereiches an der Dicke der bereits gesetzten Implantate (8) ausgerichtet wird.

7. Verfahren nach Anspruch 6,
**dadurch gekennzeichnet,**
**dass** die Dicke des Summierungsbereiches um einen Sicherheitsbereich (17) erweitert wird.

8. Verfahren nach einem der vorherigen Ansprüche,
**dadurch gekennzeichnet,**
**dass** die Volumendaten (1) mittels eines "cone beam CT" Gerätes, aufgenommen sind.

9. Verfahren nach einem der vorherigen Ansprüche,
**dadurch gekennzeichnet,**
**dass** die Panoramaschicht durch eine Abwicklung als Panoramaaufnahme in der Darstellungsebene dargeboten wird.

## Claims

1. Method for the two-dimensional presentation of volume data (1) of a jaw of a patient, wherein the two-dimensional volume data (1) are recorded by a tomographic recording equipment, wherein three-dimensional planning data are allocated to the volume data, which comprise geometrical data of at least one implant (8) to be inserted, wherein in the volume data a panorama layer (9) to be presented is defined, which is displayed as a panoramic radiograph in the plane of display,
and wherein at first a initial panorama layer (5) is defined, on the basis of which the implants and their position in the jaw are defined,
**characterized in**
**that** then starting from the initial panorama layer (5) based on the position and the geometric data of the implant the panorama layer (9) to be presented is calculated, which is placed on apical and occlusal points (10, 11) of the implants, wherein the panorama layer (9) to be presented is bent at least horizontally and vertically,
**that** the orientation of the panorama layer to be presented is automatically adjusted to a change in the position, inclination, length or diameter of an implant by an algorithm running on a computer.

2. Method according claim 1,
**characterized in**
**that** the course of the panorama layer (9) to be presented is aligned at a bent surface, which is adapted to the planning data.

3. Method according claim 1 or 2,
**characterized in**
**that** the panorama layer (9) to be presented is aligned to all virtual implants (8), which are present in the planning data.

4. Method according to any of the previous claims,
**characterized in**
**that** new implants (8) are inserted per input at the screen, in particular via mouse-click, into the panorama layer (9) to be presented or into the panorama projection.

5. Method according claim 4,
**characterized in**
**that** the position and/or the inclination of the added implants (8) are automatically aligned to a panorama layer (4), which is defined by already inserted implants (8).

6. Method according to any of the previous claims,
**characterized in**
**that** during creation of a panoramic projection, namely the mediate sum over several panorama layers on a area of summation, the thickness of an area of summation is aligned to on the thickness of the already inserted implants (8).

7. Method according claim 6,
**characterized in**
**that** the thickness of the area of summation is extended by a safety margin (17).

8. Method according to any of the previous claims,
**characterized in**
**that** the volume data (1) are recorded via a "cone beam CT"-device.

9. Method according to any of the previous claims,
**characterized in**
**that** the panorama layer is displayed by a flat projection as a panoramic radiograph in the plane of display.

## Revendications

1. Procédé de représentation bidimensionnelle de données volumiques (1) d'une mâchoire d'un patient, sachant que les données volumiques (1) sont prises au moyen d'un appareil de prise de vue tomographique, sachant que des données d'implantation tridimensionnelles sont assignées aux données volumiques, lesquelles présentent des données géométriques d'au moins un implant (8) à mettre en place, sachant qu'une couche panoramique à représenter (9) est définie dans les données volumiques, laquelle est présentée en tant que prise de vue panoramique dans le plan de représentation,
et qu'une couche panoramique initiale (5) est d'abord déterminée, à l'aide de ses implants et de leur position dans la mâchoire,
**caractérisé en ce**
**qu'**en partant de la couche panoramique initiale (5) à l'aide de la position et des données géométriques des implants, la couche panoramique à représenter (9) est calculée, qui est placée par les points apicaux et occlusifs (10, 11) des implants, sachant que la couche panoramique à représenter (9) est courbée horizontalement et verticalement au moins localement,
**que** l'alignement de la couche panoramique à représenter est adapté automatiquement à une modification de position, d'inclinaison, de longueur ou de diamètre par un algorithme fonctionnant sur un ordinateur.

2. Procédé selon la revendication 1,
**caractérisé en ce**
**que** le tracé de la couche panoramique à représenter (9) est aligné sur une surface courbe adaptée aux données d'implantation.

3. Procédé selon la revendication 1 ou 2,
**caractérisé en ce**
**que** la couche panoramique à représenter (9) est alignée sur tous les implants (8) virtuels se trouvant dans les donnés d'implantation.

4. Procédé selon l'une des revendications précédentes,
**caractérisé en ce que**
de nouveaux implants (8) sont mis en place dans la couche panoramique à représenter (9) ou dans la projection panoramique par une saisie sur l'écran, en particulier par un clic de souris.

5. Procédé selon la revendication 4,
**caractérisé en ce**
**que** la position et/ou l'inclinaison des implants (8) ajoutés est/sont alignée(s) automatiquement à la couche panoramique (4) définie par des implants (8) déjà mis en place.

6. Procédé selon l'une des revendications précédentes,
**caractérisé en ce**
**que** lorsqu'une projection panoramique est créée, c'est-à-dire la somme calculée sur plusieurs couches panoramiques sur une zone d'addition, l'épaisseur d'une zone d'addition est alignée sur l'épaisseur des implants (8) déjà mis en place.

7. Procédé selon la revendication 6,
**caractérisé en ce que**
l'épaisseur de la zone d'addition est élargie d'une zone de sûreté (17).

8. Procédé selon l'une des revendications précédentes,
**caractérisé en ce**
**que** les données volumiques (1) sont prises via un appareil « cone beam CT ».

9. Procédé selon l'une des revendications précédentes,
**caractérisé en ce**
**que** la couche panoramique est présentée par un développement en tant que prise de vue panoramique dans le plan de représentation.
